# EUROPEAN PATENT APPLICATION

(11) **EP 3 446 664 A1**
(43) Date of publication of application: **27.02.2019**
(21) Application number: 17187077.7
(22) Date of filing: 21.08.2017
(51) Int. Cl.: A61F 6/14

(54) **INTRAUTERINE DEVICE**

(71) Applicant: Kurz, Karl-Heinz, 40545 Düsseldorf (DE)
(72) Inventor: Kurz, Karl-Heinz, 40545 Düsseldorf (DE)
(74) Representative: Cohausz & Florack

(57) **Abstract**

The invention relates to an intrauterine device comprising a plastic pulling element, **characterized in that** an anti-infective agent is located on the thread and is positioned for release to the cervical canal.

## Description

### TECHNICAL FIELD OF THE INVENTION

The invention relates to an intrauterine device (IUD) with anti-infective properties effective in the cervical canal.

### BACKGROUND OF THE INVENTION

IUDs are used for insertion into the uterine cavity of a woman, in which case, for example, a contraceptive, such as a copper coil or a product which has to be absorbed slowly in the body is fitted around or placed on the IUD.

IUDs are required to be replaced periodically. Since the IUD sits in the uterine cavity and is thus not easily accessible, it is necessary to have a means of reliably, safely, and conveniently removing said IUD.

It is also known well known that a pulling element that extends to and even through the cervical canal provides for such a removal means. This has been described to be in various forms, including a wire, as described in EP 0274 794 A2, a strand, as described in US 3,407,806, and a plastic thread, as described in EP 0 455 767 B1.

In EP 0 455 767 B1, the IUD described therein contains a thread made of a plastic material with a higher fusion temperature than the plastic material of the T-shaped base, such as nylon or polypropylene, acting as a pulling element. This single thread runs along the entire length of the vertical section of the IUD and is described to provide a means to securely incorporate a single thread into an IUD without the use of knots or wholes and by which means sources of infection are greatly reduced.

WO 97/28826 A2 describes contraceptive release systems with antibacterial and/or antiviral activity. The contraceptive release system is selected from an IUD, a vaginal ring, and an intracervical system comprising a combination of an organic compound with hormonal contraceptive activity and an organic compound with antibacterial and/or antiviral activity. This combination is either incorporated in the contraceptive release system or applied thereon.

### SUMMARY OF THE INVENTION

The problem underlying the invention resides in the provision of an IUD that reduces the risk of sexually transmitted infections.

The invention provides an intrauterine device comprising a pulling element made of a first plastic material, wherein an anti-infective agent is located on the pulling element and is positioned for release to the cervical canal.

The IUD of the invention considerably reduces the concentration of infectious species in the cervix, thereby greatly reducing the risk of or even preventing infection with a sexually transmitted disease.

### PREFERRED EMBODIMENTS OF THE INVENTION

The IUD of the invention can be structured such as commonly available IUDs. In general, the IUD of the invention may have a T-shaped base, Y-shaped base or any other form that is suited for the purpose of an IUD. In the case, an IUD is essentially T-shaped or Y-shaped, it comprises a transverse section(s) and a vertical section. The transverse section serves to hold the IUD in place in the uterine cavity after insertion. The vertical section of the "T" or "Y" shape in use of the IUD extends caudally (i.e. towards the cervical canal).

The IUD of the invention contains at least one pulling element. The pulling element is made of a smooth flexible plastic material. The pulling element may comprise a single or two threads. The pulling element is securely attached to the T-shaped base or Y-shaped base of the IUD.

The IUD of the invention comprises an anti-infective agent which is located on the pulling element of the IUD of the invention. The term "located on" as used herein when referring to the anti-infective agent means that the anti-infective agent is applied to or incorporated into the pulling element. In certain embodiments of the invention, the anti-infective agent is coated onto the pulling element, wherein the coat may be along the entire length of the pulling element, along the length of the portion of the pulling element to be positioned in the cervical cavity, or located on only one or more specific portions of the pulling element. Alternatively, the anti-infective agent may be threaded into or onto the pulling element. In all embodiments, at least a portion of the anti-infective agent is to be positioned for release within the cervical canal.

As used herein the term "anti-infective agent" refers to an agent with antibacterial, antiviral and/or antifungal activity. Thus, the anti-infective agent is a germicidal agent. The anti-infective agent comprised in the IUD of the invention can be each anti-infective agent that exerts anti-infective activity against infective species and is used in therapy. Preferably, the anti-infective agent has the ability to considerably decrease the concentration of sexually transmitted infectious species in the cervix, and/or to prevent an infection. The anti-infective agent used according to the invention is a metal that provides anti-infective metal ions. Thus, the anti-infective agent of the invention can be selected from copper, iron, silver, anti-infective alloys thereof, and gold alloys.

According to another embodiment the anti-infective agent can be also be an organic anti-infective agent used to treat infectious conditions in the human body in addition to the aforementioned anti-infective agent. In this case the anti-infective agent can be applied from the pulling element of the IUD of the invention in any manner suitable for release to the cervical canal.

Preferably the anti-infective agent comprised in the IUD of the invention is copper or silver or an anti-infective metal alloy containing at least one of these metals. Copper is considered to be particularly advantageous as its risk profile for use in the human body is known from extensive use of copper as a contraceptive agent in IUDs.

In case of using copper or another spermicidal metal or metal alloy as the anti-infective agent on the pulling element of the IUD according to the invention it is believed that the contraceptive effectiveness of the IUD of the invention will be enhanced in comparison to conventional IUDs. Without being bound to a single theory, it is thought that this can be attributed to the fact that some of the sperm may find their way into the tiny ramifications of the cervical canal where they can survive for a certain period of time, protected from the copper ions of conventional IUDs, which are only locally released into the uterus, but do not reach the cervix. Providing additional copper on the pulling element comprised in the IUD of the invention, results in a local release of copper ions into the cervical canal where the copper ion can exert both their anti-infective and spermicidal effect.

The anti-infective agent located on the pulling element of the IUD can have any form that is attachable to the pulling element. It may have the form of a particle having, for example, the shape of a ball, any other more or less spherical shape, cylinder, collar or tube. It may be attached on the pulling element such as a strand of pearls. The anti-infective metals mentioned before can also be deposited on the pulling element, and may encircle the pulling element. In case an anti-infective organic compound is used as an additional anti-infective agent it may also be deposited on the pulling element or may be contained in a reservoir which is attached to the pulling element.
**Fig. 1** shows an embodiment of the IUD of the invention.
**Fig. 2** shows the position of an IUD according to the invention inserted in the user.

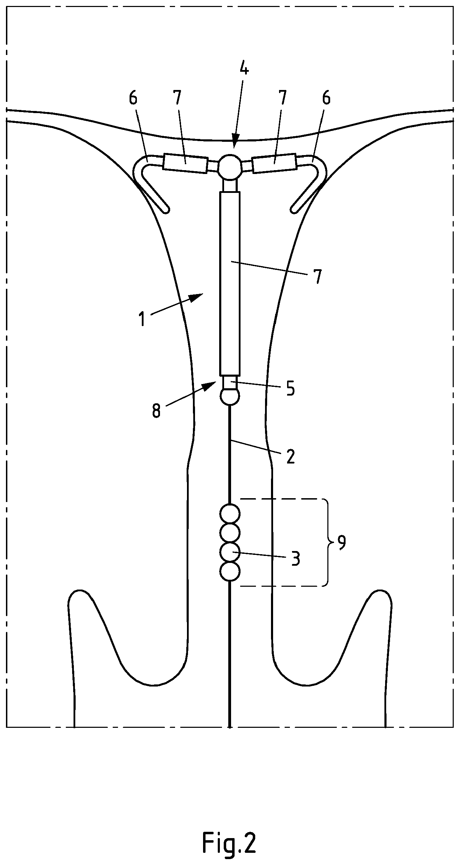

In the T-shaped embodiment, the base of the IUD typically may comprise one transverse section extending across the uterine cavity along the left-right body axis as depicted in Fig. 2 and/or along the anterior-posterior axis in case of movement of the device following insertion. The transverse section is in connection with the vertical section, which extends along the uterine cavity along the rostral-caudal body axis. Typically the vertical section bisects the transverse section.

In the Y-shaped embodiment, the base of the IUD typically may comprise two transverse sections, each connected to and extending from the vertical section of the IUD base at an angle, as depicted in Fig. 2. The skilled person knows that the T-shaped or Y-shaped base may be slightly modified, but essentially maintains the general form of a "T" and "Y", respectively. For example, the radial ends of the transverse section(s) may be curved in order to improve device safety, as is commonly known in the art.

Typically, the T-shaped or Y-shaped base of the IUD is formed from a second plastic material. Typically, transversal and vertical sections of the IUD can be formed from a plastic material as one single piece. Suitable second plastic materials are those plastics which are usually taken for the manufacture of IUD and which provide the desired flexibility of the IUD. Flexible in this context means that the IUD can be bent in order to be easily positioned in and easily removed from the cervical canal. Preferably the plastic material used for the T-shaped or Y-shaped base element of the IUD is polyethylene or a polyethylene copolymer. However, any plastic material that hitherto has been used for the manufacture of the IUD base can be used in the invention.

The IUD of the invention comprises a contraceptive agent which is located on the vertical and/or the transverse section(s) of the IUD. In all cases, the contraceptive agent is positioned on the IUD for release into the uterine cavity.

Preferably, the contraceptive agent is positioned along the transverse and vertical sections of the T-shaped and/or Y-shaped base of the IUD. Such a positioning is known to have a higher contraceptive effectiveness compared to IUDs where the contraceptive agent is positioned only along the vertical section of the IUD base. This is attributed to the fact that an IUD where the contraceptive is only positioned along the length of the middle leg of the T-shaped element may not effectively exercise its spermicidal activity on the lateral passageway of the sperm in the uterus on its way to the fallopian tubes. By the additional positioning of a contraceptive along the transverse section(s) of the T-shaped or Y-shaped base of the IUD, the concentration of the contraceptive agent is effectively extended to the lateral passageways of the sperm, thereby preventing essentially all sperm from reaching the fallopian tubes.

The contraceptive agent positioned along the vertical and/or transverse section(s) of the T-shaped or Y-shaped element can be any known contraceptive agent. The contraceptive is released in the amount necessary to exercise its contraceptive activity. It goes without saying that the contraceptive can be released using any known release system. The release of the contraceptive can be continuous or periodic.

The contraceptive agent comprised in the IUD of the invention can be any known hormone and/or any spermicidally active agent commonly used. Preferably the spermicidally active agent is copper or a copper alloy.

Preferably, the copper is selected from is present in the form of a wire arranged in the form of a coil or a collar around a part of the T-shaped base or Y-shaped base. Alternatively, hormones having a contraceptive effect can be used as the contraceptive agent in the IUD of the invention.

As mentioned above, the IUD of the invention comprises at least one pulling element that extends to and preferably through the cervical cavity. The pulling element may be attached to the vertical and/or transverse section(s) of the base of the IUD. For example, the pulling element(s) comprised in the IUD of the invention can be fastened at the free end of the vertical section of the IUD such that when the IUD is inserted, the pulling element is positioned at least partially in the cervical canal. The terms "cervix", "cervical canal", and "neck of the uterus" are used interchangeably. The pulling element in all cases serves to permit the easy removal of the IUD from the uterine cavity. The terms "uterus" and "uterine cavity" are used interchangeably.

In a preferred embodiment of the invention, the pulling element runs along the entire length of vertical section of the IUD, wherein the vertical section of the IUD base encircles at least a portion of the pulling element within the uterine cavity.

The pulling element comprised in the IUD according to the invention can be of any plastic material that is compatible with the human body. Providing an IUD with a pulling element made of plastic material reduces possible sources of infection, as the plastic pulling element does not allow for substantial surface adhesion of infectious species. Using a plastic material for the pulling element of the IUD of the invention reduces the risk of infection. Fixing the pulling element by casting section(s) of the T-shaped or Y-shaped element around the pulling element is advantageous as it further reduces the risk of infection. In a particularly preferred embodiment of the invention, section(s) of the T-shaped or Y-shaped element are cast around section(s) of the pulling element.

The first plastic material used for the manufacture of the pulling element is strong enough not to break or rip apart when pulling the pulling element to remove the IUD from the uterus. The pulling element may be made of the same plastic material that is used for the vertical and/or transverse section(s) of the IUD base. The pulling element may be alternatively made of a different plastic material that is used for the vertical and/or transverse section(s) of the IUD base. In a preferred embodiment, the pulling element is made of a different plastic material than that which is used for the vertical and/or transverse section(s) of the IUD base. In a particularly preferred embodiment, the pulling element is made of a plastic material selected from the group consisting of polyamide, for example nylon, polypropylene, polyester, and polyurethane, or any combination thereof.

It is particularly preferred that a single pulling element is used. It is particularly preferred that a thread is used as the pulling element. It is particularly preferred that a single thread is used as the pulling element comprised in the IUD of the invention. The adhesion surface of a single thread made of plastic material provides fewer possibilities for infectious species to adhere to it by reducing the total adhesion surface for infectious species.

The dosage of the anti-infective agent that is released can be adapted in such a way so as to ensure a sufficiently high local concentration of the anti-infective agent in the cervix to effectively exercise its anti-infective activity, i.e. to effectively reduce or eliminate infectious species in the cervix. The release of the anti-infective agent can be continuous or periodic.

One advantage of a modulated release system is that the cervical cavity is not unnecessarily exposed to the anti-infective agent in an effort to avoid developing resistance against said agent.

As used herein the term "infectious species" refers to bacteria, viruses or fungi, in particular such bacteria, viruses or fungi transmitting sexually transmittable diseases.

In the following the invention will further be explained with references to the drawings.

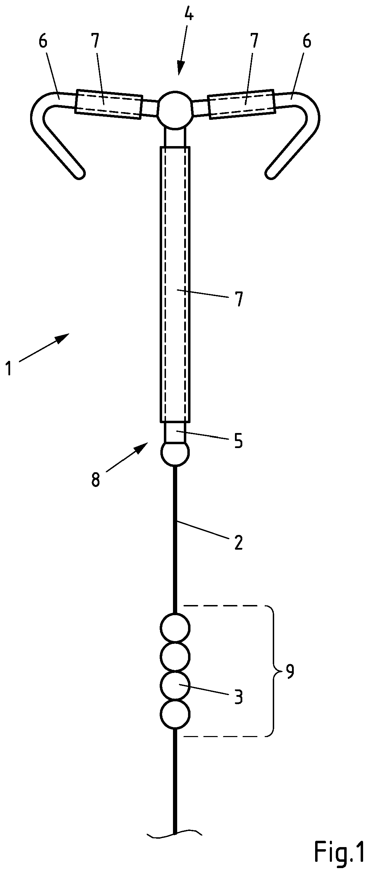

Fig. 1 shows an exemplary IUD 1 according to the invention comprising a pulling element made of a plastic material 2 fastened on the IUD 1, an anti-infective agent or component 3 is located on pulling element 2, such that it is positioned for release to the cervical cavity. The IUD as illustrated in Fig. 1 comprises a T-shaped element 4 with a vertical section 5 and two transverse sections 6. The plastic pulling element 2 runs along the entire length of vertical section 5 of the T-shaped element 4. Since the pulling element 2 runs along the entire length, it is wedged by the plastic of the vertical section 5 of the IUD base, which is cast around the pulling element. The T-shaped element 4 is positioned in the uterus, while the two transverse sections 6 of the T-shaped element 4 serve to hold the IUD in place within the uterine cavity. A contraceptive agent 7 is positioned along the length of the vertical section 5 and along the length of the vertical sections 6 of the T-shaped element 4, in the form of a coil. The pulling element 2 projects from the lower end 8 of the vertical section 5 of the IUD. The anti-infective agent 3 surrounds the pulling element at least a portion of the pulling element 9 so that it is situated in the cervical canal. The two transverse sections 6 are curved into the direction of the vertical section 5.

Fig. 2 shows a schematic representation of the placement of an IUD according to the invention in the uterus of a user. Here the pulling element 2 projects caudally from the lower end 8 from the IUD and runs from there through the uterine cavity and the cervical canal into the vagina.

## Claims

1. An intrauterine device comprising a pulling element made of a first plastic material, **characterized in that** an anti-infective agent is located on the pulling element and is positioned for release to the cervical canal.

2. The intrauterine device according to claim 1, wherein it comprises a T-shaped base or Y-shaped base made of a second plastic material to which the pulling element is attached.

3. The intrauterine device according to any of claim 1 or 2, wherein a contraceptive agent is positioned on the vertical and/or on the at least one transverse section of the T-shaped base or Y-shaped base.

4. The intrauterine device according to any of claims 2 or 3, wherein the transverse section/sections are curved towards the vertical section of the T-shaped or Y-shaped element.

5. The intrauterine device according to any one of claims 1 to 4, wherein the anti-infective agent is selected from the group consisting of copper, iron, silver, gold, and anti-infective alloys, or anti-infective combinations thereof.

6. The intrauterine device according to any one of claims 2 to 5, wherein the second plastic material is a flexible plastic material, preferably a polyethylene polymer or a polyethylene copolymer.

7. The intrauterine device according to any one of claims 1 to 6, wherein the first plastic material of the pulling element is selected from the group consisting of polyamide, nylon, polypropylene, and polyurethane.

8. The intrauterine device according to any one of claims 3 to 7, wherein the contraceptive agent is selected from the group consisting of hormones or spermicidally active agents.

9. The intrauterine device according to claim 8, wherein the spermicidally active agent is copper or silver.

10. The intrauterine device according to claim 8, wherein the hormone comprises levonorgestrel.
